# EUROPÄISCHE PATENTSCHRIFT

(11) **EP 1 659 955 B1**
(45) Veröffentlichungstag und Bekanntmachung des Hinweises auf die Patenterteilung: **17.10.2007**
(21) Anmeldenummer: 04764613.8
(22) Anmeldetag: 30.08.2004
(51) Int. Cl.: A61B 17/02

(54) **HYDRAULISCHE BÄNDERSPANNVORRICHTUNG**
HYDRAULIC LIGAMENT-TENSIONING DEVICE
DISPOSITIF HYDRAULIQUE DE TENSION DE LIGAMENTS

(30) Priorität: 04.09.2003 DE 10340885; 20.10.2003 DE 10348585
(43) Veröffentlichungstag der Anmeldung: 31.05.2006
(73) Patentinhaber: Mathys AG Bettlach, 2544 Bettlach (CH)
(72) Erfinder: FANKHAUSER, Christoph, CH-4500 Solothurn (CH); DELFOSSE, Daniel, CH-3303 Jegenstorf (CH); SALZGEBER, Beat, CH-3930 Visp (CH); CLAUSEN, Bernhard, CH-1971 Grimisuat (CH); MOREILLON, Alain, CH-1971 Grimisuat (CH)
(74) Vertreter: Körfer, Thomas
(86) Internationale Anmeldenummer: PCT/EP2004/009645
(87) Internationale Veröffentlichungsnummer: WO 2005/023120

(56) Entgegenhaltungen:
- WO-A-00/78225
- WO-A-03/003951
- DE-U- 20 310 289
- FR-A- 2 840 189
- US-A- 6 022 377

## Beschreibung

Die Erfindung betrifft eine Bänderspannvorrichtung für Skelettteile, insbesondere Gelenke des menschlichen oder tierischen Körpers nach dem Oberbegriff des Anspruchs 1.

Aus der WO 00/78225 A1 ist eine Bänderspannvorrichtung für nicht-kugelige Gelenke bekannt. Die darin beschriebene Vorrichtung zum Spannen von Bändern an nicht-kugeligen Gelenken am menschlichen oder tierischen Körper umfaßt einen prismatischen, zylindrischen oder plattenförmigen Grundkörper mit einer rechten Pratze und einer linken Pratze, deren Auflageflächen parallel auf die gelenkseitige Oberfläche eines ersten an ein nicht-kugeliges Gelenk angrenzenden Knochens zur Anlage bringbar sind. Die Bänderspannvorrichtung hat einen rechten Handgriff und einen linken Handgriff, einen rechten Spannhebel und einen linken Spannhebel mit zweiten Auflageflächen, welche parallel zu den ersten Auflageflächen angeordnet sind, wobei zwischen den jeweiligen Auflageflächen des rechten Spannhebels und der rechten Pratze eine Spannweite Y und zwischen den jeweiligen Auflageflächen des linken Spannhebels und der linken Pratze dieselbe oder eine andere Spannweite X einstellbar ist. Die zweiten Auflageflächen sind auf die gelenkseitige Oberfläche eines zweiten an das Gelenk angrenzenden Knochens zur Anlage bringbar. Weiterhin umfaßt die Vorrichtung einen rechten Bedienungshebel und einen linken Bedienungshebel, welche gleichzeitig mit dem Halten der Vorrichtung mit je einer Hand am entsprechenden Handgriff einzeln mit der jeweils selben Hand betätigbar sind und eine rechte Parallelverschiebevorrichtung und eine linke Parallelverschiebevorrichtung, welche je durch den entsprechenden Bedienungshebel antreibbar sind und so mit je einem Spannhebel verbunden sind, daß bei einer Bewegung der Bedienungshebel die Spannweiten X bzw. Y unabhängig voneinander einstellbar sind. Die Parallelverschiebevorrichtungen sind als Viergelenk-Hebelgetriebe ausgebildet.

Nachteilig an der aus der WO 00/78225 A1 bekannten Bänderspannvorrichtung ist insbesondere, daß das Gelenk bzw. die Gelenkkapsel während der Anspannung der Ligamente (Bänder) zwecks Insertion der Bänderspannvorrichtung teilweise offen bleiben muß. Dies führt dazu, daß sich einige Ligamente nicht in ihrer physiologischen (natürlichen) Position befinden. Dadurch werden Messungen der Bänderspannung, welche für die korrekte Einstellung beispielsweise bei der Einbringung von Prothesen in das Gelenk nötig sind, verfälscht, was z. B. in zu starker oder zu schwacher Spannung nach der Einbringung der Prothese und postoperativen Komplikationen resultieren kann.

Die WO-A-03/003951 offenbart eine Bänderspannvorrichtung, welche die Merkmale des einleitenden Teils des Anspruchs 1 aufweist.

Der Erfindung liegt demnach die Aufgabe zugrunde, eine Bänderspannvorrichtung zu schaffen, die es ermöglicht, die Kapsel-Bandstrukturen eines prothetisch zu versorgenden Skelettteils, insbesondere Gelenkes, in natürlicher Position der Ligamente und Weichteilstrukturen anzuspannen.

Die Aufgabe wird durch die Merkmale des Anspruchs 1 gelöst.

Erfindungsgemäß erfolgt die Spannung der Ligamente dabei über eine Bänderspannvorrichtung mit einer hydraulischen Stellvorrichtung. Dadurch läßt sich ein Kraft-Weg-Diagramm während der Operation vermessen, so daß die dabei gewonnenen Erkenntnisse in den weiteren Operationsablauf, insbesondere die Auswahl der Prothesenkomponenten, einfließen können. Der Druck des Hydraulikmediums ist dabei ein Maß für die Kraft. Das zugeführte Volumen des Hydraulikmediums ist dabei ein Maß für den Weg.

Die Bänderspannvorrichtung weist dabei eine erste und eine zweite Anlageplatte auf, welche an den jeweiligen Skelettteilen in Anlage gebracht werden und zueinander durch die hydraulische Stellvorrichtung in proximal-distaler Richtung verschieblich sind.

Weiterhin sind die Anlageplatten durch einen Grundkörper miteinander verbunden, in welchem die hydraulische Stellvorrichtung angeordnet ist.

Weitere vorteilhafte Ausgestaltungen der Erfindung sind in den Unteransprüchen gekennzeichnet.

Besonders vorteilhaft ist die bewegliche Anordnung der zweiten Anlageplatte gegenüber der ersten Anlageplatte über eine anterio-posteriore Achse, welche eine Verkippung der zweiten Anlageplatte in Varus-Valgus-Richtung erlaubt.

Die glatte Oberfläche der zweiten Anlageplatte erlaubt vorteilhafterweise eine freie Bewegung des Femurknochens je nach Stellung des Gelenks und somit eine unverspannte Lage der Ligamente.

Vorteilhafterweise ist die Krafteinleitung auf die hydraulische Stellvorrichtung über ein Hydraulikmedium, dessen Druck durch eine bekannte Antriebseinheit mit einem Schrittmotor stufenlos regelbar ist, in einfacher Weise möglich.

Die Erfindung wird im folgenden anhand schematischer Darstellungen in verschiedenen Perspektiven näher erläutert.

Es zeigen:
- Fig. 1A: eine schematische, perspektivische Ansicht eines Ausführungsbeispiels einer erfindungsgemäßen Bänderspannvorrichtung in einer Ansicht von schräg oben,
- Fig. 1B: eine schematische, perspektivische Ansicht des in Fig. 1A dargestellten Ausführungsbeispiels einer erfindungsgemäßen Bänderspannvorrichtung in einer Ansicht von schräg unten,
- Fig. 1C: eine schematische Ansicht des in Fig. 1A dargestellten Ausführungsbeispiels einer erfindungsgemäßen Bänderspannvorrichtung in einer Ansicht von vorne,
- Fig. 1D: eine schematische geschnittene Ansicht des in Fig. 1A dargestellten Ausführungsbeispiels einer erfindungsgemäßen Bänderspannvorrichtung in einer Ansicht von schräg oben auf die Schnittfläche und
- Fig. 2A-C: schematische Ansichten und Schnitte einer für das in Fig. 1 dargestellte Ausführungsbeispiel einer erfindungsgemäßen Bänderspannvorrichtung geeigneten Antriebseinheit.

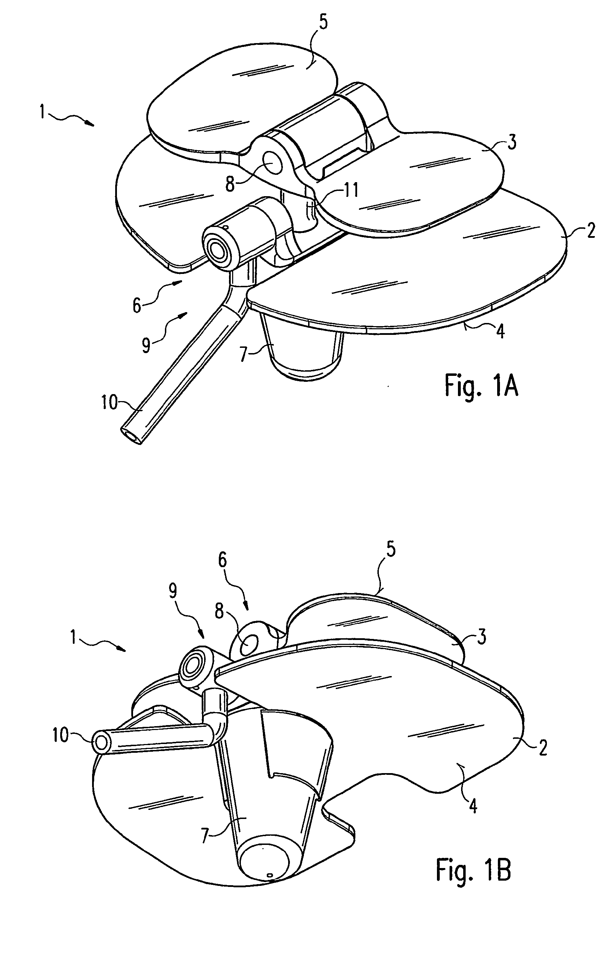

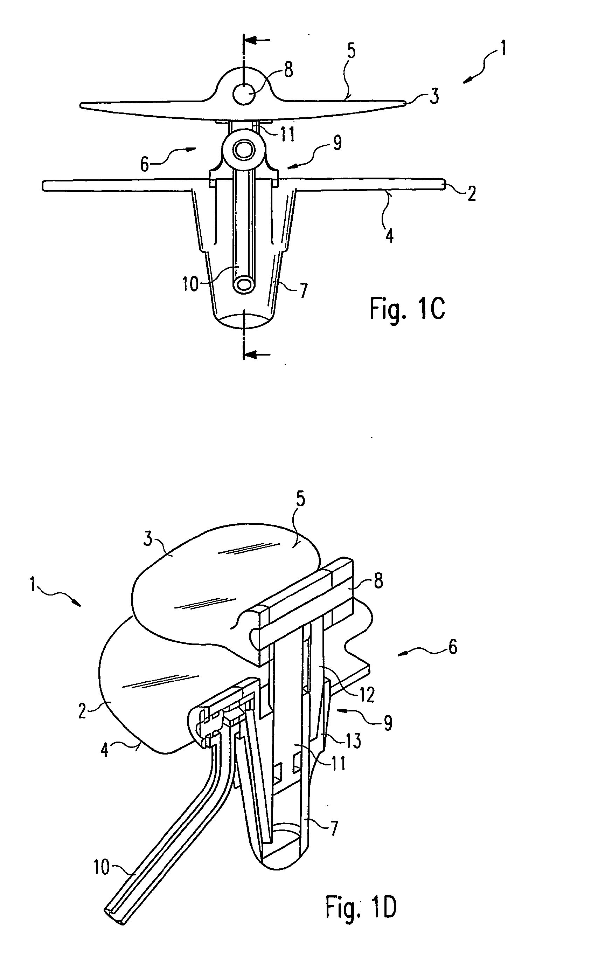

Fig. 1A bis 1C zeigen in verschiedenen schematischen, perspektivischen Gesamtansichten ein Ausführungsbeispiel einer erfindungsgemäß ausgestalteten Bänderspannvorrichtung 1 für ein Kniegelenk, welche hydraulisch verstellbar ist.

Die Bänderspannvorrichtung 1 umfaßt dabei eine erste, distale Anlageplatte 2 und eine zweite, proximale Anlageplatte 3. Die Anlageplatten 2, 3 sind dabei symmetrisch zweiteilig ausgebildet und so ausgeformt, daß sie in Anlage an nicht weiter dargestellte Skelettteile gebracht werden können. Im beschriebenen Ausführungsbeispiel sind dies die Skelettteile eines Kniegelenks.

Die erste, distale Anlageplatte 2 weist eine distale tibiale Anlagefläche 4 auf, welche an den Knochenschnitt des Schienbeins (Tibia) anliegt. Sie ist flächig ausgebildet und weist eine der Form des Schienbeinkopfes angepaßte Außenkontur auf, welche vorzugsweise randseitig bündig mit dem Schienbeinkopf abschließt.

Entsprechend weist die zweite, proximale Anlageplatte 3 eine proximale femurale Anlagefläche 5 auf, welche an den Kondylen des Oberschenkels (Femur) anliegt. Diese kann entsprechend der rundlichen Form der Kondylen eine angepaßte, ebenfalls gerundete Kontur aufweisen. Die Anlagefläche 5 ist dabei glatt oder sogar poliert, um eine annähernd reibungsfreie Bewegung des Femurs an der Anlagefläche zu ermöglichen. Die zweite, proximale Anlageplatte 3 ist dabei kleiner als die erste, distale Anlageplatte 2, da die Anlagefläche der Femurkondylen kleiner ist als die Anlagefläche des Schienbeinkopfes.

Die Anlageplatten 2, 3 sind durch einen Grundkörper 6 miteinander verbunden, welcher einen Verankerungszapfen 7 aufweist. Mittels des Verankerungszapfens 7 wird die Bänderspannvorrichtung 1 in der Tibia verankert. Zu diesem Zweck wird bei der vorbereitenden Bearbeitung des Schienbeinkopfes in diesen eine Knochenkavität eingebracht, welche im weiteren Operationsverlauf bei der prothetischen Versorgung eines Kniegelenks den tibialen Teil einer Kniegelenksprothese aufnimmt.

Die erste, distale Anlageplatte 2 ist dabei an dem Grundkörper 6 fixiert, während die zweite, proximale Anlageplatte 3 mit dem Grundkörper 6 über eine Achse 8 verbunden ist, welche eine Verkippung der zweiten, proximalen Anlageplatte 3 gegenüber der ersten, distalen Anlageplatte 2 ermöglicht. Die Kippbewegung findet dabei in der Frontalebene in Varus-Valgus-Richtung statt. Dadurch kann sich das Gelenk frei und unverspannt trotz gespannter Bänderspannvorrichtung 1 bewegen, wenn die Knochen relativ zueinander gebeugt werden. Dies ist insbesondere in Hinblick auf die Vorbereitung der prothetischen Versorgung eines Gelenks wichtig, da die Gesamtsituation der ligamentären Strukturen im Bereich des Gelenks, ihre Anzahl und Spannung vor der Implantation einer Prothese untersucht werden muß.

Weiterhin umfaßt der Grundkörper 6 erfindungsgemäß eine hydraulische Stellvorrichtung 9, welche eine Veränderung der Lage der Anlageplatten 2, 3 zueinander in proximal-distaler Richtung erlaubt. Die hydraulische Stellvorrichtung 9 umfaßt, wie aus Fig. 1D ersichtlich, eine Zuleitung 10, durch welche das Hydraulikmedium zugeleitet wird. Als Hydraulikmedium ist vorzugsweise ein biokompatibles Fluid wie z.B. isotonische Kochsalzlösung vorgesehen, da diese bei einer eventuellen Leckage oder beim Bruch der Zuleitung 10 keine Probleme im Operationsfeld verursacht.

Das Hydraulikmedium wird unter hohem Druck in die Zuleitung 10 gepreßt und betätigt einen in der hydraulischen Stellvorrichtung 9 in dem Verankerungszapfen 7 angeordneten Kolben 11, welcher mit der zweiten, proximalen Anlageplatte 3 in Wirkverbindung steht. Um beim Aufspreizen der Anlageplatten 2, 3 eine korrekte Führung zu erhalten, ist ein Führungsstift 12 vorgesehen, welcher sich parallel zum Kolben 11 erstreckt und in einer Ausnehmung 13 des Grundkörpers 6 geführt ist. Der Kolben 11 wirkt bei Beaufschlagung mit Druck auf die Achse 8 ein und verschiebt dadurch die zweite, proximale Anlageplatte 3 in proximaler Richtung. Wird der Druck durch das Hydraulikmedium abgebaut, bewegt sich die zweite, proximale Anlageplatte 3 durch die Spannung der Ligamente in distaler Richtung zurück.

Der für die Beaufschlagung der hydraulischen Bänderspannvorrichtung 1 benötigte Druck wird von einem nicht näher dargestellten Hydraulikaggregat über eine Antriebseinheit 14 zur Verfügung gestellt.

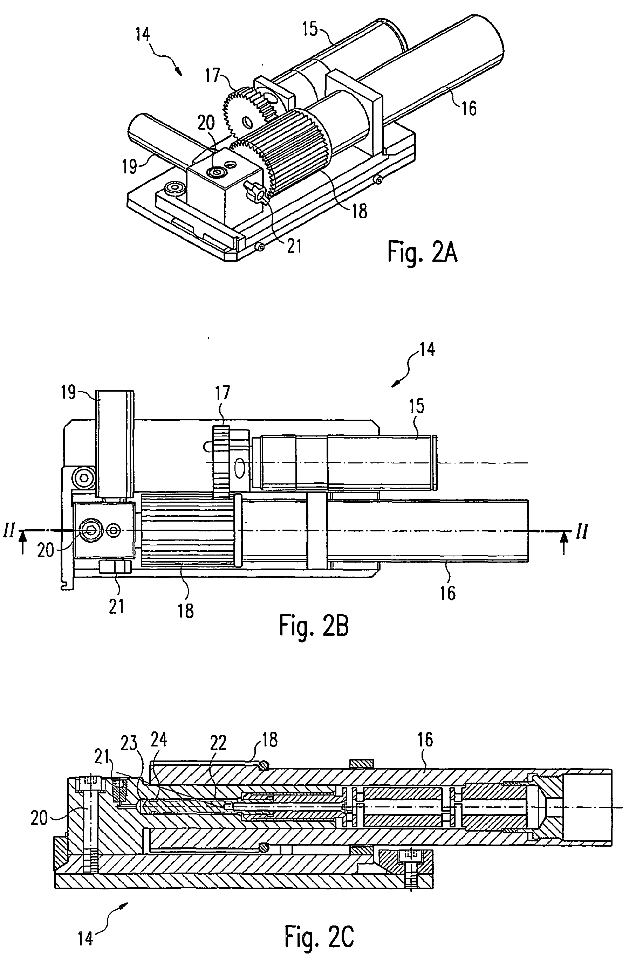

Die Antriebseinheit 14 ist in den Fig. 2A bis 2C in verschiedenen Ansichten dargestellt. Erläutert werden nur die erfindungswesentlichen Komponenten. Die Antriebseinheit 14 kann im übrigen in bekannter Weise aufgebaut sein.

Ein Schrittmotor 15 ist dabei parallel zu einer Welle 16 des Hydraulikaggregats angeordnet. Ein Zahnrad 17 des Schrittmotors 15 greift in eine Verzahnung 18 der Welle 16 ein. Durch die Rotation des Zahnrads 17 des Schrittmotors 15 wird die Welle 16 des Hydraulikaggregats angetrieben.

Weiterhin sind ein Drucksensor 19 für den in der Hydraulikleitung 10 herrschenden Druck sowie ein Entlüftungsventil 20 vorgesehen. Die Hydraulikleitung 10 ist mittels eines geeigneten Anschlusses 21 mit der Antriebseinheit 14 verbunden und führt zu der Bänderspannvorrichtung 1.

Wie aus der geschnittenen Ansicht in Fig. 2C ersichtlich, welche einen Schnitt durch die in Fig. 2B dargestellte Antriebseinheit 14 entlang der mit II-II bezeichneten Linie darstellt, wird durch die Rotation der Welle 16 des Hydraulikaggregates ein in der Welle 16 angeordneter Stift 22 in einer Achsrichtung der Welle 16 bewegt. Dadurch wird der Druck in einem mit Hydraulikmedium gefüllten Volumen 23 je nach Drehrichtung erhöht oder verringert. Ein oder mehrere Dichtringe 24 verhindern das Austreten des Hydraulikmediums. Wie bereits weiter oben erwähnt, eignet sich als Hydraulikmedium ein biokompatibles Fluid wie beispielsweise isotonische Kochsalzlösung oder jede andere antiseptische Spüllösung.

Durch den stufenlosen Antrieb durch den Schrittmotor 15 kann der Druck in dem mit Hydraulikmedium gefüllten Volumen 23 beliebig und stufenlos erhöht oder verringert werden und die Bänderspannvorrichtung 1 dadurch beliebig aufgespannt oder entlastet werden. Durch den Drucksensor 19 und/oder durch die Einbindung der erfindungsgemäß ausgestalteten Bänderspannvorrichtung 1 können die Druckwerte in geeigneter Weise dokumentiert werden.

Die Dokumentation der mit einem bestimmten Druckwert und damit mit einem definierten Weg des Kolbens 11 der Bänderspannvorrichtung 1 sowie einer definierten Verlängerung der Ligamente verbundenen Kraft ermöglicht die Aufnahme eines Kraft-Weg-Diagramms z. B. für das prothetisch zu versorgende Gelenk in einem Flexionsbereich zwischen 0° und ca. 120°. Um eine optimale Anpassung des Ligamenteapparates an die zu implantierende Prothese bzw. an die nach der prothetischen Versorgung etablierte Situation zu ermöglichen, muß sich die durch die Ligamente ausgeübte Zugkraft über den Flexionsbereich konstant verhalten.

Mittels der erfindungsgemäßen Bänderspannvorrichtung 1 kann ein dynamisches Kraft-Weg-Diagramm erstellt werden.

Herkömmliche Bänderspannvorrichtungen 1 ermöglichen einerseits nur die statische Aufspreizung des Gelenks unter einem definierten Flexionswinkel, andererseits sind die Ligamente durch das Anbringen der Bänderspannvorrichtung 1 so aus ihrer natürlichen Lage verschoben, daß eine Einstellung der Bänderspannung bei applizierter Bänderspannvorrichtung 1 unmöglich ist.

Im Gegensatz dazu ist durch die erfindungsgemäß ausgestaltete Bänderspannvorrichtung 1 sowohl eine Flexion des Gelenks bei applizierter Bänderspannvorrichtung 1 möglich, da die glatte oder polierte Anlagefläche 5 der zweiten Anlageplatte 3 eine freie Anpassung der Knochenposition gemäß der jeweiligen Felxionslage ermöglicht, andererseits befinden sich die Ligamente bedingt durch die kompakte Form und die hydraulische Betätigung der Bänderspannvorrichtung 1 in ihrer natürlichen Lage, da das Gelenk bzw. der Bänder-Kapsel-Apparat nach dem Einsetzen der Bänderspannvorrichtung 1 bis auf eine kleine Öffnung, durch welche die Zuleitung 10 geführt ist, vollständig geschlossen werden kann, so daß die präoperative Situation bis auf kleine Abweichungen wieder herstellbar ist. Nach der prothetischen Versorgung des Gelenks können dann einzelne Ligamente direkt im notwendigen Umfang gelöst werden, um eine optimale Beweglichkeit des Gelenks über den gesamten Flexionsbereich zu erzielen.

Die erfindungsgemäß ausgestaltete Bänderspannvorrichtung 1 läßt durch ihre besondere Form und Betätigung eine detaillierte Vorbereitung auf die prothetische Versorgung mit optimaler Annäherung an die Situation eines intakten Gelenks zu.

Die erfindungsgemäße Bänderspannvorrichtung kann nicht nur an Gelenken, sondern auch an anderen Skelettteilen, beispielsweise auch zum Spreizen von Wirbelkörpern bei Bandscheibenoperationen zum Einsatz kommen.

## Patentansprüche

1. Bänderspannvorrichtung (1) zur Aktivierung des Band- und/oder Kapselapparates während der Implantierung eines Skelettimplantats mit einer ersten, distalen Anlageplatte (2) zur Anlage an einem ersten Skelettteil und einer zweiten, proximalen Anlageplatte (3) zur Anlage an einem zweiten Skelettteil, wobei die erste, distale Anlageplatte (2) gegenüber der zweiten, proximalen Anlageplatte (3) durch eine hydraulische Stellvorrichtung (9) verschieblich ist,
**dadurch gekennzeichnet,**
**daß** die erste Anlageplatte (2) und die zweite Anlageplatte (3) durch einen Grundkörper (6) miteinander verbunden sind, in welchem die hydraulische Stellvorrichtung (9) der Bänderspannvorrichtung (1) angeordnet ist.

2. Bänderspannvorrichtung nach Anspruch 1,
**dadurch gekennzeichnet,**
**daß** die erste Anlageplatte (2) fest mit dem Grundkörper (6) verbunden ist.

3. Bänderspannvorrichtung nach Anspruch 2,
**dadurch gekennzeichnet,**
**daß** die zweite Anlageplatte (3) über eine Achse (8) beweglich mit dem Grundkörper (6) verbunden ist.

4. Bänderspannvorrichtung nach Anspruch 3,
**dadurch gekennzeichnet,**
**daß** die zweite Anlageplatte (3) gegenüber der ersten Anlageplatte (2) medial-lateral verkippbar angeordnet ist.

5. Bänderspannvorrichtung nach einem der Ansprüche 1 bis 4,
**dadurch gekennzeichnet,**
**daß** an dem Grundkörper (6) ein tibialer Verankerungszapfen (7) ausgebildet ist.

6. Bänderspannvorrichtung nach einem der Ansprüche 1 bis 5,
**dadurch gekennzeichnet,**
**daß** die hydraulische Stellvorrichtung (9) einen in dem Grundkörper (6) und dem Verankerungszapfen (7) angeordneten Kolben (11) aufweist.

7. Bänderspannvorrichtung nach Anspruch 6,
**dadurch gekennzeichnet,**
**daß** der Kolben (11) in Wirkverbindung mit der zweiten, proximalen Anlageplatte (3) steht.

8. Bänderspannvorrichtung nach Anspruch 6 oder 7,
**dadurch gekennzeichnet,**
**daß** ein zu dem Kolben (11) paralleler Führungsstift (12) in einer Ausnehmung (13) des Grundkörpers (6) geführt ist.

9. Bänderspannvorrichtung nach einem der Ansprüche 1 bis 8,
**dadurch gekennzeichnet,**
**daß** die hydraulische Stellvorrichtung (9) eine Zuleitung (10) aufweist.

10. Bänderspannvorrichtung nach einem der Ansprüche 1 bis 9,
**dadurch gekennzeichnet,**
**daß** die hydraulische Stellvorrichtung (9) mit einem Hydraulikmedium gefüllt ist.

11. Bänderspannvorrichtung nach Anspruch 10,
**dadurch gekennzeichnet,**
**daß** als Hydraulikmedium eine biokompatible Lösung dient.

12. Bänderspannvorrichtung nach Anspruch 10 oder 11,
**dadurch gekennzeichnet,**
**daß** als Hydraulikmedium isotone Kochsalzlösung dient.

13. Bänderspannvorrichtung nach einem der Ansprüche 10 bis 12,
**dadurch gekennzeichnet,**
**daß** der Druck des Hydraulikmediums auf den Kolben (11) durch eine Antriebseinheit (14) stufenlos regelbar ist.

14. Bänderspannvorrichtung nach Anspruch 13,
**dadurch gekennzeichnet,**
**daß** die Antriebseinheit (14) einen Schrittmotor (15) aufweist.

15. Bänderspannvorrichtung nach Anspruch 14,
**dadurch gekennzeichnet,**
**daß** der Schrittmotor (15) über ein Zahnrad (17) und eine Verzahnung (18) eine Welle (16) eines Hydraulikaggregats antreibt.

16. Bänderspannvorrichtung nach Anspruch 15,
**dadurch gekennzeichnet,**
**daß** in der Welle (16) ein Stift (22) angeordnet ist, durch dessen axiale Verschiebung eine stufenlose Vergrößerung oder Verkleinerung eines das Hydraulikmedium enthaltenden Volumens (23) erfolgt.

17. Bänderspannvorrichtung nach einem der Ansprüche 13 bis 16,
**dadurch gekennzeichnet,**
**daß** die Antriebseinheit (14) einen Drucksensor (19) aufweist.

18. Bänderspannvorrichtung nach einem der Ansprüche 13 bis 17,
**dadurch gekennzeichnet,**
**daß** die Antriebseinheit (14) einen Anschluß (21) für ein Hydraulikleitung (10) aufweist.

19. Bänderspannvorrichtung nach einem der Ansprüche 1 bis 18,
**dadurch gekennzeichnet,**
**daß** die proximale Anlagefläche (5) der zweiten, proximalen Anlageplatte (3) eine glatte und/oder polierte Oberfläche aufweist.

## Claims

1. Ligament tensioning device (1) for activating the ligamentous and/or capsular apparatus while implanting a skeletal implant, with a first, distal bearing plate (2) for bearing against a first skeletal part and a second, proximal bearing plate (3) for bearing against a second skeletal part, wherein the first, distal bearing plate (2) can be displaced with respect to the second, proximal bearing plate (3) by a hydraulic actuating device (9),
**characterised in**
**that** the first bearing plate (2) and the second bearing plate (3) are connected together by a base body (6), in which the hydraulic actuating device (9) of the ligament tensioning device (1) is disposed.

2. Ligament tensioning device according to Claim 1,
**characterised in**
**that** the first bearing plate (2) is connected to the base body (6) in a fixed manner.

3. Ligament tensioning device according to Claim 2,
**characterised in**
**that** the second bearing plate (3) is connected to the base body (6) in a mobile manner via a pivot pin (8).

4. Ligament tensioning device according to Claim 3,
**characterised in**
**that** the second bearing plate (3) is disposed so as to be tiltable medially-laterally with respect to the first bearing plate (2).

5. Ligament tensioning device according to any one of Claims 1 to 4,
**characterised in**
**that** a tibial anchoring pin (7) is formed on the base body (6).

6. Ligament tensioning device according to any one of Claims 1 to 5,
**characterised in**
**that** the hydraulic actuating device (9) comprises a
plunger (11) which is disposed in the base body (6) and the anchoring pin (7).

7. Ligament tensioning device according to Claim 6,
**characterised in**
**that** the plunger (11) is actively connected to the second, proximal bearing plate (3).

8. Ligament tensioning device according to Claim 6 or 7,
**characterised in**
**that** a guide pin (12) which is parallel to the plunger (11) is guided in a recess (13) of the base body (6).

9. Ligament tensioning device according to any one of Claims 1 to 8,
**characterised in**
**that** the hydraulic actuating device (9) comprises a feed line (10).

10. Ligament tensioning device according to any one of Claims 1 to 9,
**characterised in**
**that** the hydraulic actuating device (9) is filled with a hydraulic medium.

11. Ligament tensioning device according to Claim 10,
**characterised in**
**that** a biocompatible solution serves as the hydraulic medium.

12. Ligament tensioning device according to Claim 10 or 11,
**characterised in**
**that** isotonic sodium chloride solution serves as the hydraulic medium.

13. Ligament tensioning device according to any one of Claims 10 to 12,
**characterised in**
**that** the pressure of the hydraulic medium on the plunger (11) can be steplessly adjusted by a drive unit (14).

14. Ligament tensioning device according to Claim 13,
**characterised in**
**that** the drive unit (14) comprises a stepping motor (15).

15. Ligament tensioning device according to Claim 14,
**characterised in**
**that** the stepping motor (15) drives a shaft (16) of a hydraulic unit via a gear wheel (17) and a tooth system (18).

16. Ligament tensioning device according to Claim 15,
**characterised in**
**that** a pin (22) is disposed in the shaft (16), through the axial displacement of which pin a volume (23) containing the hydraulic medium is steplessly enlarged or diminished.

17. Ligament tensioning device according to any one of Claims 13 to 16,
**characterised in**
**that** the drive unit (14) comprises a pressure sensor (19).

18. Ligament tensioning device according to any one of Claims 13 to 17,
**characterised in**
**that** the drive unit (14) comprises a connection (21) for a hydraulic line (10).

19. Ligament tensioning device according to any one of Claims 1 to 18,
**characterised in**
**that** the proximal bearing face (5) of the second, proximal bearing plate (3) has a smooth and/or polished surface.

## Revendications

1. Dispositif de tension de ligaments (1) pour l'activation de l'appareil ligamentaire et/ou capsulaire pendant l'implantation d'un implant du squelette avec une première plaque d'appui distale (2) pour l'application sur une première partie de squelette et une deuxième plaque d'appui proximale (3) pour l'application sur une seconde partie de squelette, dans lequel la première plaque d'appui distale (2), en opposition à la seconde plaque d'appui proximale (3), peut être déplacée par un dispositif d'ajustement hydraulique (9),
**caractérisé en ce que**
la première plaque d'appui (2) et la seconde plaque d'appui (3) sont reliées entre elles par un corps de base (6), dans lequel est agencé le dispositif d'ajustement hydraulique (9) du dispositif de tension de ligaments (1).

2. Dispositif de tension de ligaments selon la revendication 1,
**caractérisé en ce que**
la première plaque d'appui (2) est raccordée solidement au corps de base (6).

3. Dispositif de tension de ligaments selon la revendication 2,
**caractérisé en ce que**
la seconde plaque d'appui (3) est raccordée par un axe (8) mobile au corps de base (6).

4. Dispositif de tension de ligaments selon la revendication 3,
**caractérisé en ce que**
la seconde plaque d'appui (3) est agencée en opposition à la première plaque d'appui (2), de façon à pouvoir basculer dans le plan médian latéral.

5. Dispositif de tension de ligaments selon l'une des revendications 1 à 4,
**caractérisé en ce que**
sur le corps de base (6), est configuré un tenon d'ancrage de tibia (7).

6. Dispositif de tension de ligaments selon l'une des revendications 1 à 5,
**caractérisé en ce que**
le dispositif d'ajustement hydraulique (9) présente un piston (11) disposé dans le corps de base (6) et le tenon d'ancrage (7).

7. Dispositif de tension de ligaments selon la revendication 6,
**caractérisé en ce que**
le piston (11) se trouve en liaison efficace avec la seconde plaque d'appui proximale (3).

8. Dispositif de tension de ligaments selon la revendication 6 ou 7,
**caractérisé en ce que**
un pivot de guidage (12) parallèle au piston (11) est guidé dans un évidement (13) du corps de base (6).

9. Dispositif de tension de ligaments selon l'une des revendications 1 à 8,
**caractérisé en ce que**
le dispositif d'ajustement hydraulique (9) présente une conduite d'amenée (10).

10. Dispositif de tension de ligaments selon l'une des revendications 1 à 9,
**caractérisé en ce que**
le dispositif d'ajustement hydraulique (9) est rempli d'un agent hydraulique.

11. Dispositif de tension de ligaments selon la revendication 10,
**caractérisé en ce que**
en tant qu'agent hydraulique, on utilise une solution biocompatible.

12. Dispositif de tension de ligaments selon la revendication 10 ou 11,
**caractérisé en ce que**
en tant qu'agent hydraulique, on utilise une solution d'eau salée isotone.

13. Dispositif de tension de ligaments selon l'une des revendications 10 à 12,
**caractérisé en ce que**
la pression de l'agent hydraulique sur le piston (11) peut être régulée en continu au moyen d'une unité d'entraînement (14).

14. Dispositif de tension de ligaments selon la revendication 13,
**caractérisé en ce que**
l'unité d'entraînement (14) comporte un moteur pas à pas (15).

15. Dispositif de tension de ligaments selon la revendication 14,
**caractérisé en ce que**
le moteur pas à pas (15) entraîne, par l'intermédiaire d'une roue dentée (17) et d'une denture (18), un arbre (16) d'un ensemble hydraulique.

16. Dispositif de tension de ligaments selon la revendication 15,
**caractérisé en ce que**
un pivot (22) est agencé dans l'arbre (16) par le déplacement axial duquel on obtient une augmentation progressive ou une diminution d'un volume (23) contenant l'agent hydraulique.

17. Dispositif de tension de ligaments selon l'une des revendications 13 à 16,
**caractérisé en ce que**
l'unité d'entraînement (14) présente un capteur de pression (19).

18. Dispositif de tension de ligaments selon l'une des revendications 13 à 17,
**caractérisé en ce que**
l'unité d'entraînement (14) présente un raccordement (21) pour une conduite hydraulique (10).

19. Dispositif de tension de ligaments selon l'une des revendications 1 à 18,
**caractérisé en ce que**
la surface d'appui proximale (5) de la seconde plaque d'appui proximale (3) présente une surface lisse et/ou polie.
